# DEMANDE DE BREVET EUROPEEN

(11) **EP 0 692 226 A1**
(43) Date de publication de la demande: **17.01.1996**
(21) Numéro de dépôt: 95401622.6
(22) Date de dépôt: 05.07.1995
(51) Int. Cl.: A61F 2/06, A61F 2/36

(54) **Prothèse interne et dispositif prothétique correspondant**

(30) Priorité: 12.07.1994 FR 9408666
(71) Demandeur: LABORATOIRE PEROUSE IMPLANT, F-60540 Bornel (FR)
(72) Inventeur: Perouse, Eric, F-95290 L'Isle Adam (FR)
(74) Mandataire: Jacobson, Claude

(57) **Abrégé**

Cette prothèse interne (3) est munie d'un réseau d'irrigation (9).

Elle se met en place avec une chambre implantable (5) et avec un cathéter (6) adapté pour relier cette dernière au réseau d'irrigation.

Application aux prothèses artérielles textiles, aux endoprothèses expansibles et aux prothèses rigides orthopédiques.

## Description

La présente invention est relative aux prothèses internes.

De nombreux types de prothèses internes sont utilisés en chirurgie, par exemple des prothèses tubulaires souples, en matériau textile, destinées à raccorder deux bouts d'artère, des prothèses expansibles ou "stents", destinées à soutenir un tronçon d'artère après dilatation, ou encore des prothèses orthopédiques rigides.

En considérant par exemple le cas des prothèses tubulaires souples de chirurgie vasculaire, on constate parfois l'apparition d'une infection à l'emplacement de la prothèse. La prothèse infectée doit être retirée rapidement, et l'expérience montre qu'il n'est alors plus possible de la remplacer par une autre prothèse synthétique, car l'infection réapparaît.

Par suite, à l'heure actuelle, la seule solution, en cas d'infection, permettant de sauver la vie du malade est l'allogreffe, qui se heurte malheureusement à une insuffisance de donneurs.

L'invention a pour but de remplacer une prothèse infectée par une autre prothèse sans recourir à l'allo-greffe.

A cet effet, l'invention a pour objet une prothèse interne, caractérisée en ce qu'elle est munie d'un réseau d'irrigation, notamment réparti sur toute l'étendue de la prothèse.

La prothèse suivant l'invention peut comporter une ou plusieurs des caractéristiques suivantes:
- le réseau comprend un réseau de conduits capillaires d'irrigation;
- le réseau de conduits capillaires communique avec un canal d'alimentation s'étendant à partir du corps de la prothèse;
- le canal forme une excroissance d'un revêtement extérieur du corps de la prothèse, ce revêtement étant notamment constitué d'un polymère souple, en particulier à base de silicone;
- lorsque le corps de la prothèse comprend un tube souple en matière textile, les conduits capillaires débouchent dans la lumière intérieure de la prothèse;
- des conduits capillaires additionnels traversent l'épaisseur du tube et débouchent sur la partie textile de la prothèse;
- lorsque la prothèse est une endoprothèse expansible, le réseau d'irrigation comprend au moins une hélice souple constituée d'un tube capillaire creux perforé vers l'extérieur de la prothèse; et
- lorsque la prothèse est une prothèse rigide d'orthopédie,le réseau d'irrigation comprend un réseau de rainures communicantes prévu sur la surface de la prothèse.

L'invention a également pour objet un dispositif prothétique comprenant :
- une prothèse interne telle que définie ci-dessus;
- une chambre implantable; et
- un cathéter adapté pour relier la chambre implantable au réseau d'irrigation.

Dans un mode de réalisation de ce dispositif, dans le cas où la prothèse comporte un canal d'alimentation, le cathéter peut être relié à l'extrémité libre du canal au moyen d'un raccord.

Des exemples de réalisation de l'invention vont maintenant être décrit en regard des dessins annexés, sur lesquels :
- la Figure 1 est un schéma général montrant l'implantation d'un dispositif prothétique conforme à l'invention;
- les Figures 2 à 4 illustrent schématiquement trois étapes de réalisation d'une prothèse interne conforme à l'invention;
- la Figure 5 est un schéma montrant la prothèse de la Figure 4 en coupe transversale;
- la Figure 6 est une coupe longitudinale, à grande échelle, du raccord utilisé dans le dispositif de la Figure 1;
- la Figure 7 est une vue schématique en perspective d'une endoprothèse expansible conforme à l'invention;
- la Figure 8 est une vue en élévation d'une prothèse de hanche conforme à l'invention.

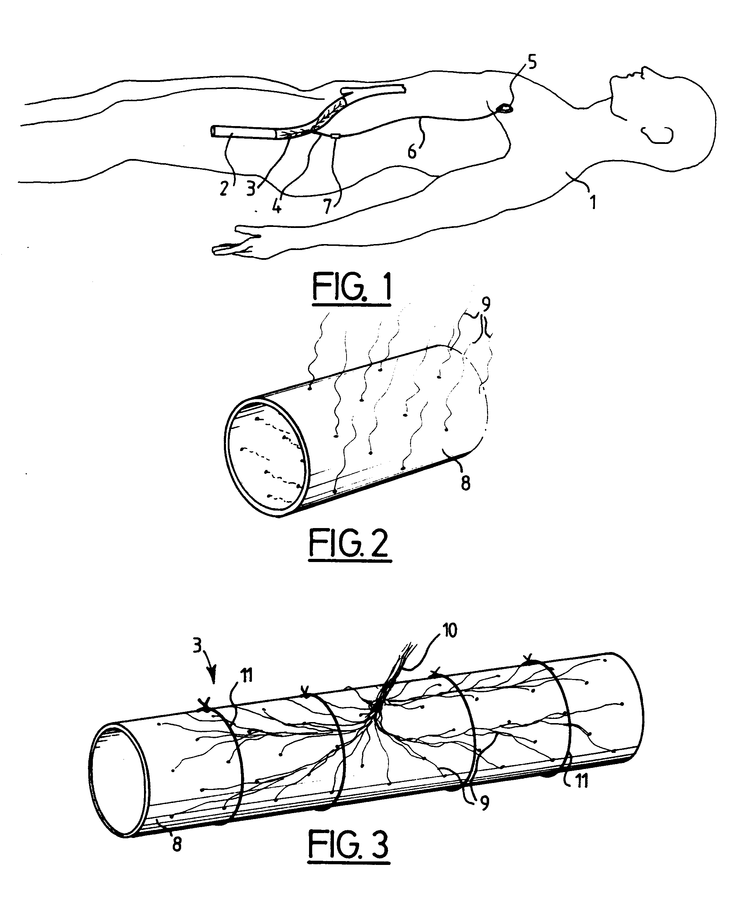

On a représenté à la Figure 1 le contour d'un patient 1 dont l'artère fémorale 2 a été, sur une certaine longueur, remplacée par une prothèse 3 conforme à l'invention, munie d'un canal d'alimentation 4.

Sous la peau du patient, dans la région de son thorax, on a disposé une chambre implantable 5 d'où part un cathéter 6. L'extrémité de ce cathéter opposée à la chambre implantable 5 est reliée de façon étanche au canal 4 par un raccord 7.

Comme il est bien connu dans la technique, une chambre implantable est un petit réservoir dont la face extérieure comporte un septum ou membrane en silicone destiné à recevoir de multiples piqûres, lesquelles sont effectuées à l'aide d'une aiguille spéciale dite à pointe de Huber. Un exemple de chambre implantable est commercialisé sous la marque POLYSITE.

On décrira maintenant la fabrication de la prothèse 3 en regard des Figures 2 à 4.

On part d'un tube souple en matière textile 8 (Figure 2), dans lequel on pique un grand nombre de filaments ou fibres 9, dépassant tous vers l'extérieur. Le tube 8 est typiquement en fibres de polyester tissées ou tricotées, et les fibres 9 en polytétrafluoroéthylène (PTFE). Les fibres 9 sont piquées de manière homogène dans le tube 8, sur toute l'étendue de celui-ci.

Ensuite, les fibres 9 sont peignées sur la surface extérieure du tube 8 en direction d'un point unique de la surface extérieure de celui-ci, jusqu'à former une mèche 10 en ce point (Figure 3). Quelques liens 11 entourant le tube 8 assurent le maintien provisoire des fibres sur celui-ci.

L'ensemble ainsi obtenu est monté sur un mandrin, puis immergé dans une solution de trempage d'un mélange de résine à base de silicone non réticulée, et de xylène, servant de solvant. Après avoir effectué plusieurs trempages, on obtient un revêtement extérieur d'une épaisseur suffisante, et on procède à la réticulation de la résine par chauffage.

On obtient ainsi un tube 8 portant sur sa surface extérieure un réseau de fibres 9 enrobées par la résine, ainsi que la mèche 10, également enrobée d'un conduit de résine 12, mais avec l'extrémité des fibres dépassant hors de ce conduit.

En saisissant l'extrémité libre des fibres qui n'adhèrent ni au polyester ni à la résine, on les extrait du conduit 12 et du revêtement de résine, de sorte que le conduit 12 devient un canal libre dont l'extrémité distale communique avec autant de conduits capillaires qu'il y avait de fibres 9. Les extrémités des conduits capillaires débouchent donc sur la partie textile de la prothèse.

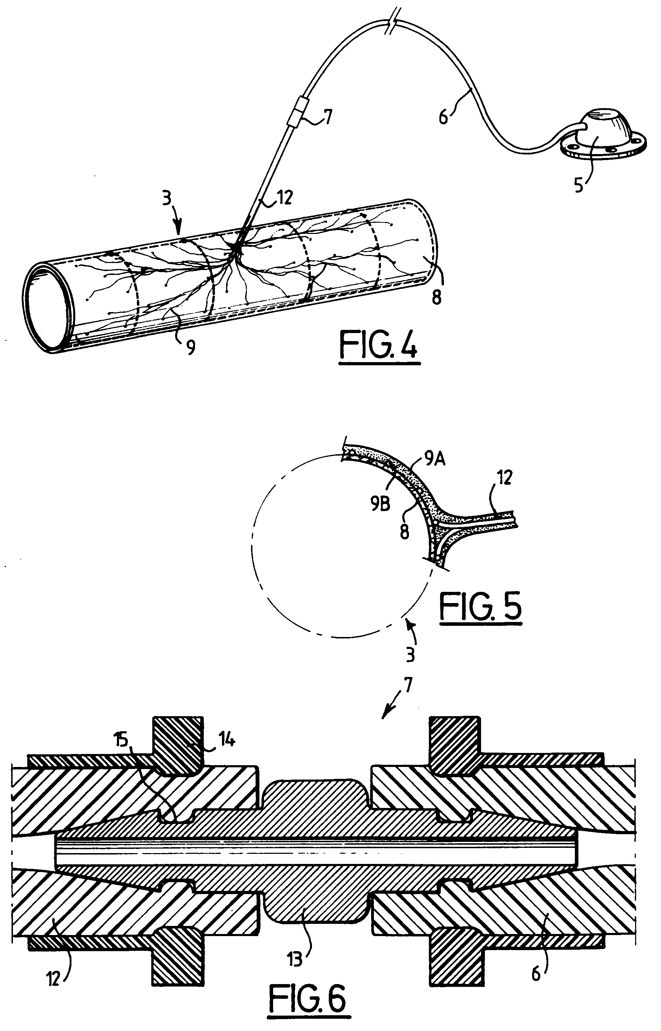

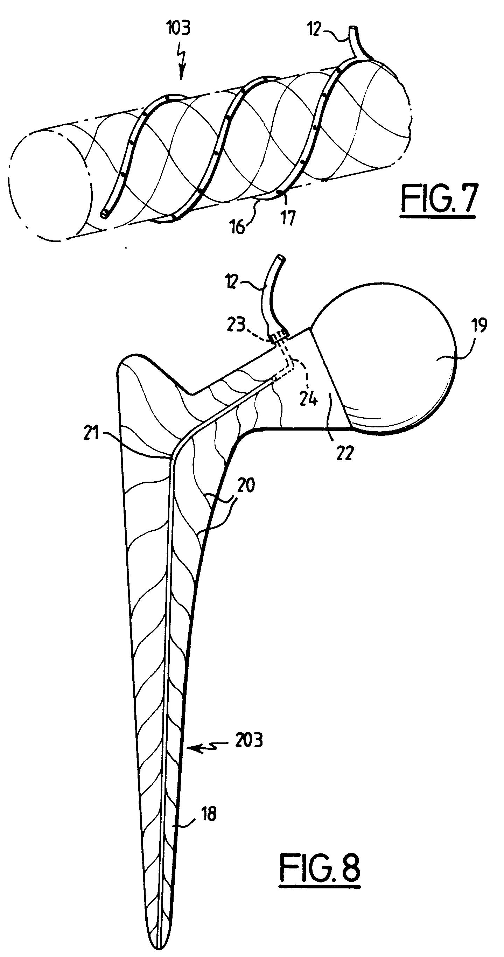

On obtient ainsi la prothèse schématisée aux Figures 4 et 5, dans laquelle le tube 8 porte sur toute sa surface extérieure une couche de résine 9A qui délimite un réseau de conduits capillaires d'irrigation 9B partant de la base d'un canal d'alimentation 12. Ce dernier peut être relié de façon étanche à l'extrémité distale du cathéter 6 au moyen du raccord 7, lequel, comme représenté à la Figure 6, comprend un double embout de raccordement 13 sur lequel s'emboîte à force, de chaque côté, l'extrémité adjacente du canal 12 et du cathéter 6, ces derniers étant maintenus sur le raccord 13 au moyen de bagues de maintien 14 qui en pressent une partie d'extrémité dans une gorge respective 15 du raccord.

La résine de revêtement précitée est à la fois biocompatible et souple, de façon à conférer à la prothèse une certaine souplesse.

L'ensemble 3 à 7 représenté aux Figures 1 et 4 est utilisé lorsque le patient a développé une infection à l'emplacement d'une prothèse classique précédemment implantée. Après avoir retiré la prothèse infectée, on met en place la nouvelle prothèse 3 ainsi que la chambre implantable 5, et on injecte dans cette dernière un produit antibiotique adapté.

Ce produit parvient au canal 12 via le cathéter 6 et, de là, il imprègne par capillarité l'ensemble des conduits capillaires 9B, et donc l'ensemble du tissu constituant le tube 8. Ceci évite l'apparition d'une nouvelle infection.

L'ensemble 3 à 7 décrit ci-dessus peut également être utilisé pour délivrer dans la région de la prothèse des facteurs de croissance servant à réendothélialiser la prothèse, ou encore pour délivrer localement dans la région de la prothèse des substances thrombolysantes ou anti-thrombotiques ou, plus généralement, favorisant la circulation sanguine. Cette dernière application permet d'envisager la mise en place de prothèses sur des artères qui se thrombosent facilement, comme l'artère coronaire.

En variante, certaines fibres 9 peuvent être exclues de l'opération de peignage et sortir du revêtement de résine. Ces fibres sont ensuite coupées puis extraites, après réticulation de la résine, et permettent ainsi d'irriguer également l'extérieur de la prothèse 3.

En variante également, on peut remplacer les filaments 9 par des filaments creux restant en place dans la résine.

La Figure 7 illustre l'application de l'invention a une endoprothèse expansible ou "stent" 103. Cette endoprothèse est constituée par un ensemble d'hélices élastiques entrecroisées, typiquement en acier inoxydable. Suivant l'invention, l'une au moins de ces hélices est constituée d'un tube capillaire 16 dont une extrémité constitue le canal d'alimentation 12 et qui présente, sur toute sa longueur, des orifices 17 tournés radialement vers l'extérieur. L'autre extrémité du tube est obstruée.

Ainsi, en reliant le canal 12 à la chambre implantable, le produit de traitement diffuse dans l'artère, de façon homogène, dans la région de l'endo-prothèse, ce qui produit un effet d'irrigation du vaisseau analogue à celui obtenu au moyen des fibres de PTFE dans la réalisation des Figures 1 à 6.

La Figure 8 illustre l'application de l'invention à une prothèse rigide d'orthopédie 203, dans cet exemple une prothèse fémorale comportant essentiellement une tige 18 destinée à être emboîtée dans le canal médullaire du fémur, et un cotyle sphérique 19 destiné à coopérer avec une rotule complémentaire (non représentée).

Dans ce cas, un réseau de rainures capillaires 20 est prévu, par exemple usiné, sur toute la surface de la tige 18, toutes ces rainures communiquant avec au moins une rainure principale 21. De plus, dans la partie 22 de raccordement de la tige 18 au cotyle 19, il est prévu un bossage 23 sur lequel se fixe l'extrémité distale du canal d'alimentation 12, lequel est un tube souple rapporté. Des conduits 24 sont percés à partir du bossage 23 pour relier le canal 12 à la ou à chaque rainure principale 21.

## Revendications

**1 -** Prothèse interne (3; 103; 203), caractérisée en ce qu'elle est munie d'un réseau d'irrigation (9; 16; 20, 21), notamment réparti sur toute l'étendue de la prothèse.

**2 -** Prothèse (3) suivant la revendication 1, caractérisée en ce que le réseau comprend un réseau de conduits capillaires d'irrigation (9B).

**3 -** Prothèse (3) suivant la revendication 2, caractérisée en ce que le réseau de conduits capillaires (9B) communique avec un canal d'alimentation (12) s'étendant à partir du corps (8) de la prothèse.

**4 -** Prothèse (3) suivant la revendication 3, caractérisée en ce que le canal (12) forme une excroissance d'un revêtement extérieur (9A) du corps (8) de la prothèse, ce revêtement étant notamment constitué d'un polymère souple, en particulier à base de silicone.

**5 -** Prothèse (3) suivant la revendication 4, du type dans lequel le corps de la prothèse est un tube souple (8) en matière textile, caractérisée en ce que les conduits capillaires (9B) débouchent sur la partie textile de la prothèse.

**6 -** Prothèse (3) suivant la revendication 4 ou 5, caractérisée en ce que des conduits capillaires additionnels traversent l'épaisseur du tube (8) et débouchent à l'extérieur du revêtement (9A).

**7 -** Prothèse (103) suivant la revendication 1, du type endoprothèse expansible, caractérisée en ce que le réseau d'irrigation comprend au moins une hélice souple constituée d'un tube capillaire creux (16) perforé (en 17) vers l'extérieur de la prothèse.

**8 -** Prothèse (203) suivant la revendication 1, du type prothèse rigide d'orthopédie, caractérisée en ce que le réseau d'irrigation comprend un réseau de rainures communicantes (20, 21) prévu sur la surface de la prothèse.

**9 -** Dispositif prothétique comprenant :
- une prothèse (3; 103; 203) suivant l'une quelconque des revendications 1 à 8;
- une chambre implantable (5); et
- un cathéter (6) adapté pour relier la chambre implantable au réseau d'irrigation (9; 16; 20, 21).

**10 -** Dispositif suivant la revendication 9, caractérisé en ce que, la prothèse comportant un canal d'alimentation (12), le cathéter (6) peut être relié à l'extrémité libre de ce canal au moyen d'un raccord (7).
